# EUROPEAN PATENT APPLICATION

(11) **EP 3 517 040 A1**
(43) Date of publication of application: **31.07.2019**
(21) Application number: 19152934.6
(22) Date of filing: 22.01.2019
(51) Int. Cl.: A61B 6/03, A61B 6/14

(54) **PORTABLE BITE PART FOR DETERMINING AN IMAGING AREA OF A PATIENT IN PANORAMIC, COMPUTED TOMOGRAPHY, OR CEPHALOMETRIC X-RAY IMAGING**

(30) Priority: 26.01.2018 FI 20185073
(71) Applicant: PaloDEx Group Oy, 04301 Tuusula (FI)
(72) Inventor: VARTIAINEN, Sami, FI-01620 VANTAA (FI)
(74) Representative: Berggren Oy, Helsinki & Oulu

(57) **Abstract**

The application relates to a portable bite part (230) for determining an imaging area (204) of a patient (201) in Panoramic, Computed Tomography, or Cephalometric imaging. The bite part comprises a first end (233) and a second end (234). The bite part has known dimensions. The first end is configured to be positioned in a mouth (202) of the patient. The bite part further comprises markers (231, 232). The first end is configured to indicate the area inside the mouth. The markers are configured to be positioned outside the mouth. The markers make possible to recognize positions of the markers, whereupon it is possible to calculate a location (205) of the first end with respect to an X-ray imaging unit (100) by using the positions of the markers and the known dimensions of the bite part.

## Description

### Technical field

The application relates generally to a portable bite part for determining an imaging area of a patient in Panoramic, Computed Tomography, or Cephalometric X-ray imaging.

### Background

Traditionally a patient is positioned to an X-ray imaging unit using various supporting methods that are supposed to hold a head as stationary as possible. In addition, a location of jaw is very critical with traditional methods.

Traditional supporting means are chin rest, static bite stick, and a head support where the forehead and/or temple is supported. In addition, different kind of straps are used to make the patient positioning as rigid as possible.

In addition, some imaging units have such bite sticks that are attached to the imaging unit such that attachment means allow movements of the bite sticks in some directions.

In addition, an aiming laser lines projected on patient's face are widely used. These methods give a good approximation of the location of a desired imaging area but it is still hard to know where a certain tooth is located in a unit's coordinate system.

One approach that can be considered as traditional as well is using scout images. This is a small dose panoramic image or a set of two projection images taken at 90 degrees angle that can be used as a targeting aid for a three dimensional (3D) image. This is very slow method, because it requires multiple exposures. In addition, radiation dose is slightly higher. In addition, nothing ensures that the patient does not move between the scout image and the 3D exposure.

A rigid setup is very important with this kind of approach. When the patient positioning (targeting) is done, the patient should keep steady for a whole imaging process. If the patient moves between the targeting and the imaging phases, the resulting image might be wrong. The result can be blurred image or an image of wrong area.

### Summary

One object of the invention is to provide a patient positioning system that withdraws the aforementioned drawbacks, because in an X-ray imaging it is necessary to know where an imaging area is located in a mouth of a patient.This is challenging, because the anatomy is different with different patients.

One object of the invention is fulfilled by a portable bite part, a method, a computer program, and a computer-readable medium according to the independent claims.

One embodiment is a portable bite part for determining an imaging area of a patient in Panoramic, Computed Tomography (CT), or Cephalometric X-ray imaging. The bite part comprises a first end and a second end. The bite part has known dimensions. The first end is configured to be positioned in a mouth of the patient. The bite part further comprises markers. The first end is configured to indicate the area inside the mouth. The markers are configured to be positioned outside the mouth. The markers make possible to recognize positions of the markers, whereupon it is possible to calculate a location of the first end with respect to an X-ray imaging unit by using the positions of the markers and the known dimensions of the bite part.

One embodiment is a method for using a portable bite part for determining an imaging area of a patient in Panoramic, CT, or Cephalometric X-ray imaging. The bite part comprises a first end and a second end. The bite part has known dimensions. The first end is configured to be positioned in a mouth of the patient. The method comprises steps of positioning the first end in the mouth of the patient so that a location of the first end is in the area; taking, by means of a camera part of an X-ray imaging unit, at least one image presenting the bite part, which comprises markers; and recognizing, by means of a processor part of the unit, positions of the markers from the at least one image in order to calculate the location of the first end with respect to the unit by using the positions of the markers and the known dimensions of the bite part. The first end is configured to indicate the area inside the mouth. The markers are configured to be positioned outside the mouth.

One embodiment is a computer program for determining an imaging area of a patient by means of a portable bite part in Panoramic, CT, or Cephalometric X-ray imaging, when the program is run in an X-ray imaging unit. The bite part comprises a first end and a second end. The bite part has known dimensions. The first end is configured to be positioned in a mouth of the patient. The program comprises imaging code for taking, by means of a camera part of the unit, at least one image presenting the bite part, which comprises markers; and a recognizing code for recognizing, by means of a processor part of the unit, positions of the markers from the at least one image in order to calculate the location of the first end with respect to the unit by using the positions of the markers and known dimensions of the bite part. The first end is configured to indicate the area inside the mouth. The markers are configured to be positioned outside the mouth.

One embodiment is a tangible non-volatile computer-readable medium comprising a computer program for determining an imaging area of a patient by means of a portable bite part in Panoramic, CT, or Cephalometric X-ray imaging, when the program is run in an X-ray imaging unit. The bite part comprises a first end and a second end. The bite part has known dimensions. The first end is configured to be positioned in a mouth of the patient. The program comprises an imaging code for taking, by means of a camera part of the unit, at least one image presenting the bite part, which comprises markers; and a recognizing code for recognizing, by means of a processor part of the unit, positions of the markers from the at least one image in order to calculate the location of the first end with respect to the unit by using the positions of the markers and known dimensions of the bite part. The first end is configured to indicate the area inside the mouth. The markers are configured to be positioned outside the mouth.

Further embodiments are presented in the dependent claims.

### Brief description of the figures

The embodiments are presented with reference to the following figures:
- Fig. 1a: presents an X-ray imaging unit
- Fig. 1b: presents movements of the unit
- Fig. 2a: presents a portable bite part and its positioning
- Fig. 2b: presents a use of the bite part and camera part
- Fig. 2c: presents a use of the bite part in a motion correction
- Fig. 3: presents functional parts of the unit

### Detailed description of the figures

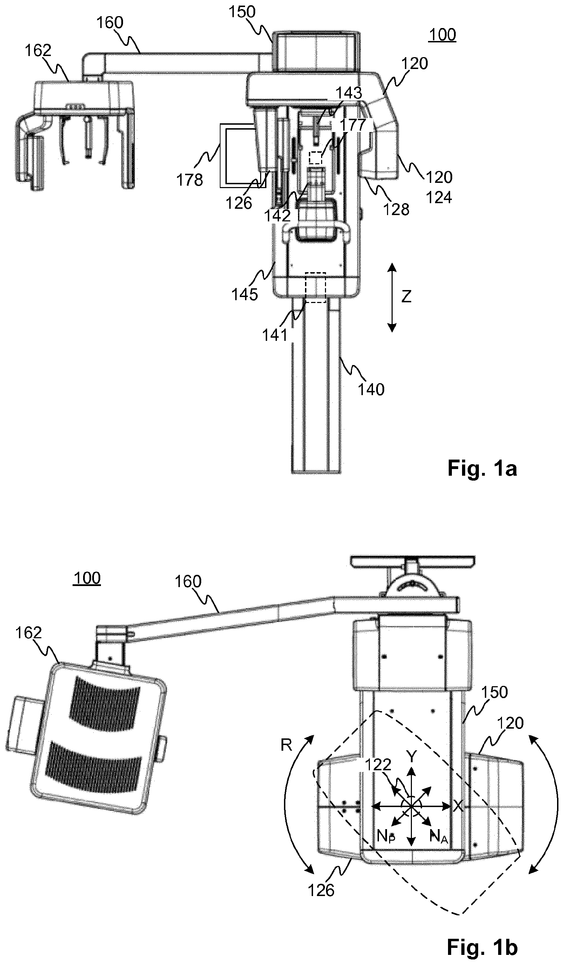

Fig. 1a presents an X-ray imaging unit 100 for imaging a determined imaging area 204 in a medical imaging.

The medical imaging can be extraoral dental imaging.

The unit 100 comprises a rotating part (rotator, gantry) 120 in order to image a Panoramic and/or CT image.

The rotating part 120 comprises an X-ray source part 124 and an X-ray imaging detector part (head) 126.

The rotating part 120 can have a form of letter C, whereupon the source part 124 can be attached on one end of the rotating part 120 and the detector part 126 can be attached on the other end of the rotating part 120.

The source part 124 can comprise an X-ray source in order to provide an X-ray beam for imaging.

The source can be common for Panoramic and CT imaging modes.

The CT imaging can be Cone beam CT (CBCT) imaging, wherein the beam is a cone-shaped beam, or alternative CT imaging, wherein the beam is a pyramidal-shaped beam, half-moon -shaped cone beam, or other shaped beam.

The detector part 126 can comprise one or two X-ray detectors in order to receive the beam and to cause the image of the area 204.

A one-detector part 126 can comprise a Panoramic detector, a Cephalometric detector, which enables also Panoramic imaging, a Panoramic/CT combination detector, a Panoramic/CT/Cephalometric combination detector, or a Panoramic/CT detector, which enables also one-shot Cephalometric imaging.

The one-detector part 126 can be adjustable so that it is possible to rotate the detector part 126 relative to the rotating part 120 in order to position its detector preferably perpendicularly to the source.

A two-detector part 126 can comprise a Panoramic detector and a CT detector, or a Cephalometric detector, which enables also Panoramic imaging, and a CT detector.

The two-detectors part 126 can be adjustable so that there are several ways to attach the detectors and it is possible to change a detector that locates within the beam. A used detector is positioned preferably perpendicularly to the source.

Alternatively, the detector part 126 can be fixed.

In addition, the rotating part 120 comprises a first collimator (X-ray beam limiting) part 128 for the source part 124 in order to collimate the beam.

The collimator part 128 can be attached in front of the source part 124 and it controls a size and shape of the beam during imaging so that the beam matches needs of a selected imaging protocol, selected image size, and related detector size.

In addition, the unit 100 comprises a column 140 in order to support the unit 100, and to adapt its height Z and simultaneously a height of the rotating part 120 to a height of a patient 201 for the Panoramic or CT imaging.

The unit 100 comprises a carriage part 145 in order to form a structure, which can provide an up/down Z-movement and a support for other parts that are adapted to be moved at the same time.

The column 140 comprises height adapting part 141 in order to cause the up/down Z-movement for the carriage part 145.

The adapting part 141 can comprise e.g. a height motor, a gear, a threaded rod, and telescopic or counterweighted part in order to realize the Z-movement as a telescopic or counterweighted movement.

The height motor drives the other parts of adapting parts 141 in order to adapt a height of the carriage 145.

In addition, the unit 100 comprises a lower shelf part 142 and a temple support part 143 in order to support the patient 201 for the Panoramic and CT imaging.

The lower shelf 142 can be attached to the carriage part 145.

The lower shelf 142 can support a tip of a chin of the patient 201 and the temple support 143 can support a forehead or temple of the patient 201.

In addition, the unit 100 comprises an upper shelf 150 in order to support the rotating part 120 and to enable the rotating part 120 to move with respect to the upper shelf 150.

The upper shelf 150 can be attached to the carriage part 145 by a fixed joint.

The rotating part 120 can be attached to the upper shelf 150 with attaching means that allow the rotating part 120 to rotate around its rotation axis 122.

The carriage 145 can comprise the lower shelf 142, the temple support 143, the upper shelf 150, and the rotating part 120, whereupon, when the height adapting part 141 realizes the Z-movement, height adapting part 141 adapts the height of the parts 142, 143, 150, 120.

Fig. 1b presents how the attaching means can allow a rotational R-movement for the rotating part 120 so that the rotating part 120 can rotate up to 400 degrees around its rotation axis 122.

The R-movement can be used for Panoramic and/or CT imaging.

In addition, the attaching means can allow a first linear Y-movement for the rotation part 120 so that its rotation axis 122 and, thus, its rotation center can be adjusted (positioned) along the Y-movement in respect to the upper shelf 150 during the imaging. The Y-movement is parallel to the upper shelf 150.

In addition, the attaching means can allow a second linear X-movement so that the rotation axis 122 can be adjusted within a plane defined by the X- and Y-movements during the imaging. The X-movement is perpendicular to the Y-movement.

In addition, the attaching means can allow a third N_{A}-movement, which moves the rotation axis 122 in respect to the rotation part 120. The N_{A}-movement of the rotation axis 122 along the beam can be used to change a magnification within the Panoramic and CT imaging modes.

In addition, the attaching means can allow a fourth N_{P}-movement, which moves the rotation axis 122 perpendicular to the beam. It can be used to a change between offset scanning and symmetrical scanning in the CT imaging, whereupon that affects the Field Of View (FOV).

In addition, the unit 100 can comprise a rotating motor part in order to rotate and/or move the rotating part 120 as presented above by the attaching means during its positioning in respect to the lower shelf 142 so that the rotating part 120 is over the lower shelf 142, and/or during irradiation.

The rotating motor part can be in the rotating part 120 or in the upper shelf 150.

In addition, the unit 100 can comprise a first moving motor part in order to move the collimator part 128 and/or the detector part 126 during positioning of the rotating part 120 and/or during scanning.

The first motor part can be in the rotating part part 120 or the upper shelf 150.

In addition, the unit 100 can comprise a Cephalometric arm part 160 and a Cephalometric head part 162 in order to provide a Cephalometric image.

The unit 100 can use the R-, X- and Y-, or X- and Y-movements during a scan phase of the Panoramic imaging resulting a Panoramic image.

In addition, the unit 100 can use the R-movement and read out the CT detector during a scan phase of the CT imaging resulting a CT image.

In addition, the unit 100 can use the X and/or Y-movements during the scan phase of the CT imaging.

The unit 100 can produce projection X-ray images of Region Of Interest (ROI) so that a center of ROI and the R-movement coincide. An effective rotation angle (aperture) can be appr. 180-360 degrees depending on the unit 100.

In addition, the unit 100 comprises a camera part 177 in order to take at least one image of a portable bite part 230 that is positioned in a mouth 202 of a patient 201.

The camera part 177 can comprise at least one camera 177.

The at least one camera 177 can be a facial optical camera 177 that is used in order to record a face of the patient 201 during a scan movement of the rotating part 120. In addition, the facial optical camera 177 can be used before or after the scan.

Alternatively, the at least one camera 177 can be a video camera 177.

The camera 177 can be mounted in a static place, e.g. in the carriage part 145 in a front of the patient 201, in the upper shelf 150, or in the rotating unit 120, where the camera 177 moves along with the rotating unit 120.

Alternatively, the at least one camera 177 can comprise at least two cameras 177.

The cameras 177 can be mounted in at least one of the followings: the carriage part 145, the upper shelf 150, and the rotating unit 120.

Alternatively, the at least one camera 177 can be an X-ray camera that comprises the X-ray source of the source part 124 and at least one detector of the detector part 126, whereupon it is possible to use the X-ray source and the detector part 126 to take at least one (X-ray) image about the bite part 230.

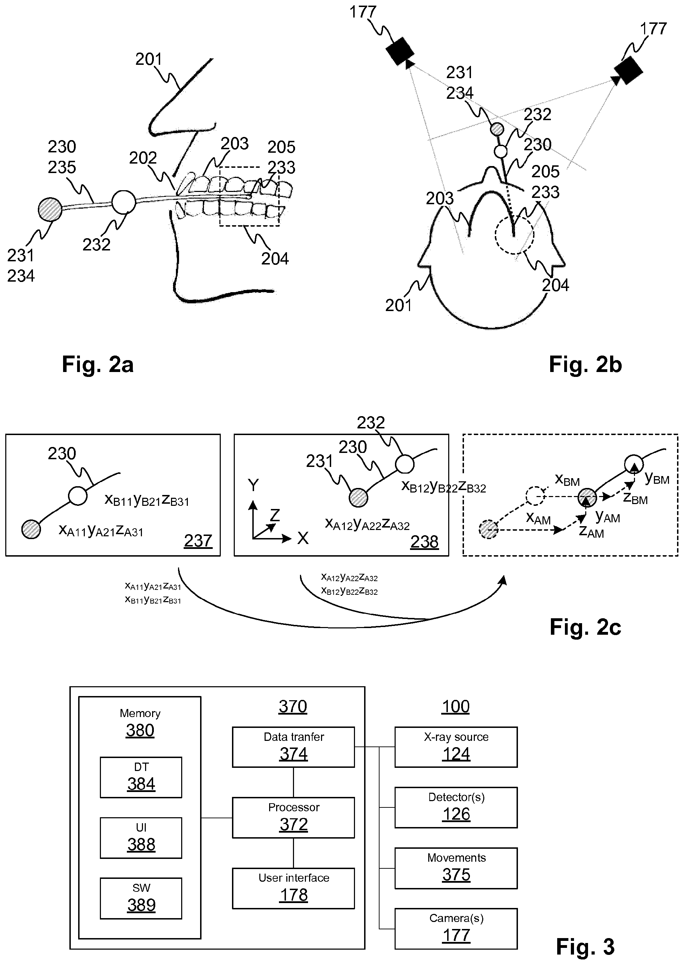

Fig. 2a presents the freely movable portable bite part 230 and how it is optically determined from at least one image, when it is positioned in e.g. the mouth 202 of the patient 201, in order to determine the area (volume) 204 for the CT imaging.

The bite part 230 can be such that there is no physical connection between the bite part 230 and the unit 100.

The bite part 230 can be e.g. a stick or other elongated part.

The bite part 230 can be straight, curved, or any other shape.

The bite part 230 comprises markers (tracking targets) 231, 232, a first end 233, a second end 234, and its known dimensions.

A material of the bite part 230 can be wood, plastic, silicon or any other radiolucent material, which is transparent to X-rays.

A material of the markers 231, 232 can be some radiopaque material, which is impenetrable to X-rays.

The dimensions of the straight bite part 230 can comprise at least one of a length of the bite part 230 (distance between the ends 233, 234), a distance between the end 233 and one marker 231, 232, a distance between the markers 231, 232, and at least one dimension of the markers 231, 232.

The dimensions of the curved bite part 230 can comprise at least one of a length of a string of the curved bite part 230 (distance between the ends 233, 234), a length of a string between the end 233 and one marker 231, 232 (distance between the end 233 and one marker 231, 232), a distance between the markers 231, 232, and at least one dimension of the markers 231, 232.

The at least dimension of the markers 231, 232 can be a diameter, shape, or size of the marker 231, 232.

The markers 231, 232 can comprise at least a first marker 231 and a second marker 232 that are designed so that it is possible to see the difference between the markers 231, 232.

The markers 231, 232 can have a spherical shape, e.g. a ball marker, or a polyhedral shape, e.g. a cube marker. Both markers 231, 232 can have the same shape or the markers 231, 232 can have different shapes.

In addition, the markers 231, 232 can have same colour or different colours, e.g. the marker 231 can be black and the marker 232 white, or vice versa.

In addition, the markers 231, 232 can be graphical symbols (features) on a surface 235 of the bite part 230. The graphical symbols can be e.g. at least one of the following symbols: square, triangle, sphere, ring, and crossing line.

In addition, the markers 231, 232 can be light emitting diodes, reflectors, or magnetic parts, which can be tracked magnetically.

The camera part 177 can comprise an ultrasound sonar system, if the markers 231, 232 are reflectors.

The camera part 177 can comprise a magnetic field detection system, if the markers 231, 232 are magnetic parts.

The markers 231, 232 can be formed to the bite part 230 so that the marker 231 locates on the end 234 and the marker 232 locates somewhere between the ends 233, 234.

Alternatively, the marker 231 can locate somewhere between the end 234 and the marker 232.

Alternatively, if the bite part 230 is curved or any other shape than straight, it cancomprise three markers 231, 232 in order to define the location of the end 233.

Fig. 2b presents the patient 201, who has been positioned into the unit 100 and supported by the parts 142, 143.

Alternatively, it is possible that the patient 201 can be positioned into the unit 100 without the parts 142, 143.

The unit 100 enables that the patient positioning does not have to be accurate. A rough positioning by the lower shelf 142 is sufficient when the bite part 230 indicates the right coordinates to the unit 100.

The straight bite part 230, which length is known, is in the mouth 202 of the patient 201 and its one end 233, which is hidden from the camera part 177, has been targeted between teeth 203 so that it locates in the area 204.

The end 233 determines an exact location of a center 205 of the area 204.

The more accurate targeting causes less X-ray exposures.

After the patient 201 has closed his/her mouth 202, the other end 234 of the bite part 230 is visible by the camera part 177 when it sticks out from the closed mouth 202.

Alternatively, the bite part 230 can be attached to the patient 201 e.g. by glue, deformable plastic, or any other suitable material.

Alternatively, the bite part 230 can be fixed to an implant or an attachment screw in the mouth 202 of the patient 201.

The end 234 can comprise at least two marker balls 231, 232, which locate in different positions along the bite part 230.

Fig. 2b presents an example of the bite part 230 that comprises two different coloured marker balls 231, 232. The marker 231 locates in the end 234 and the marker 232 locates in a appropriate distance from the marker 231 as presented in the figures.

When the sufficient dimensions (geometry) of the bite part 230 are known, it is possible to calculate the location (X,Y,Z coordinates) 205 of the hidden end 233 by knowing the locations of the markers 231, 232.

Camera models have often two parts in computer vision so that a first part models a lens distortion and a second part models projective properties as a pin-hole camera.

Such a camera model provides a many-to-one mapping of the 3D plane to a plane that represent a camera detector. In particular, photographs (images) can be undistorted with knowledge of the lens obtained through a calibration process. The resulting corrected photographs can then be considered as if they were obtained using a pin-hole camera.

Although it is not possible to invert the projective mapping in a point-wise manner, the camera model yields an inverse mapping that links each point in the detector plane with a unique line in the 3D space.

A polyhedral marker 231, 232, e.g. a cube, can be characterized by its edges. A projection maps the edges to line segments, which can be detected using a line detection algorithm, e.g. Hough transform. Lines that are parallel in the 3D space share a common vanishing point in the detector plane. In addition, vanishing points can be grouped into mutually orthogonal sets. This information can be employed in a specialized rectangle detection algorithm that recognizes the visible faces of the cubic marker 231, 232.

Spherical markers 231, 232 are projected to circles on the detector plane, whereupon a circle detection algorithm, e.g. circular Hough transform, can be used to detect the spherical markers 231, 232 .

When the geometry of the marker 231, 232 is known, it is possible to uniquely identify its 3D position by combining the scaling and position information available from a single view. The position of the marker 231, 232 in the detector plane reveals the direction in which the marker 231, 232 lies, and the scale describes the distance.

Multiple views allow the use of markers 231, 232 whose dimensions are not known a priori. In this case, points of interest are identified in each view and some kind of a descriptor is assigned to each feature. Then, matches between descriptors are sought between different views. Finally, a triangulation process is used to obtain the 3D positions of the markers 231, 232.

Features of the markers 231, 232 that have rich enough texture can be matched using image correlation techniques. Alternatively, features can be paired with descriptors, e.g. Maximally Stable Extremal Region (MSER) or Speeded-Up Robust Features (SURF). Correspondences between image pairs can then be obtained by pairing features whose descriptors have minimal distance in some metric.

The camera part 177 comprises two cameras 177, which are mounted different locations, as presented in the figure.

Each camera 177 is used to take at least one image, which presents the markers 231, 232.

The locations of the markers 231, 232 can be calculated with the known coordinates and focal length. The calculations are based on triangulation, wherein it is calculated angles where the markers 231, 232 are located in two different images, which are taken by the cameras 177.

In a two-camera part 177, for a first camera 177, a point X in the 3D space gets projected to a point on the detector plane. The projected point defines a line that goes through the focal point of the first camera 177. Similarly, a line is defined for a second camera 177.

Finally, a triangle is formed by introducing a line that goes through the focal points of the two cameras 177. Through a calibration procedure, it is possible to obtain the relative positions and orientations of the cameras 177. This allows to deduce the distance between the focal points and the angles of the triangle. Thus, in principle, trigonometry can be used to obtain the position of X, and positions of markers 231, 232.

However, in practice, the back-projected rays do not intersect and it is necessary to employ error tolerant techniques instead of the straightforward application of trigonometry.

Alternatively, if the camera part 177 comprises only one camera 177, the calculations are based on the locations of the markers 231, 232 in at least one image. A depth coordinate is calculated from the size of at least one marker 231, 232 by utilizing the fact that the closer the marker 231, 232 is, the larger it appears on the image.

The unit 100 enables to use the smaller FOV, because the location 205 of the area 204 is known better.

In addition, the smaller FOV enables to use smaller and cheaper detectors, which means cheaper units 100.

In addition, the smaller FOV means smaller x-ray dose for the patient 201.

If there is a need for reaching the teeth 203 or locations deep inside the mouth 202, the straight bite part 230 is not optimal shape for the bite part 230. Thus, the geometry of the bite part 230 requires a third marker 231, 232 in order to calculate the coordinates of the hidden end 233. With the three visible markers 231, 232, the hidden end 233 can be calculated as presented above.

Fig. 2c presents how the markers 231, 232 of the bite part 230 are used in a motion correction in the Panoramic, CT, or Cephalometric imaging.

The motion correction can be made after the imaging area 204 has been determined.

When the patient 201 is in an imaging position for the imaging and the bite part 230 is in his/her mouth 202, the source part 124 and the detector part 126 are used in order to take X-ray images 237, 238 about the desired imaging area 204.

The images 237, 238 can comprise at least a part of the patient 201 and the markers 231, 232.

The source part 124, which serves as a camera part 177, irradiates the patient 201 during scanning movements of the unit 100 in order to take the images 237, 238 and to provide image data from the images 237, 238 captured by the detector part 126.

The taken images 237, 238 can comprise at least a first X-ray image 237 and a second X-ray image 238.

First positions (coordinates) x_{A11}y_{A21}z_{A31}, x_{B11}y_{B21}z_{B31} of the markers 231, 232 can be recognized from the first image 237 and second positions x_{A12}y_{A22}z_{A32}, x_{B12}y_{B22}Z_{B32} of the markers 231, 232 from the second image 238 as above has described.

Then, a movement x_{AM}y_{AM}z_{AM}, x_{BM}y_{BM}z_{BM} of the markers 231, 232 between the first and second images 237, 238 can be detected (calculated) by means of the known positions x_{A11}y_{A21}z_{A31}, x_{B11}y_{B21}z_{B31}, x_{A12}y_{A22}z_{A32}, x_{B12}y_{B22}z_{B32} of the markers 231, 232.

The markers 231, 232 have been moved between the first and second images 237, 238 when the positions x_{A12}y_{A22}z_{A32}, x_{B12}y_{B22}z_{B32} of the second image 238 do not correspond with the positions x_{A11}y_{A21}z_{A31}, x_{B11}y_{B21}z_{B31}. of the first image 237. The movement of the markers 237, 238 means even as a movement of the patient 201 and/or the imaging area 204.

The detected movement x_{AM}y_{AM}z_{AM}, x_{BM}y_{BM}z_{BM} can be taken into account when the captured image data 237, 238, where this movement x_{AM}y_{AM}z_{AM}, x_{BM}y_{BM}z_{BM} occurs, is reconstructed to corrected X-ray images by removing, correcting, or compensating artifacts caused by the movement x_{AM}y_{AM}z_{AM}, x_{BM}y_{BM}z_{BM}.

If there is no detected movement x_{AM}y_{AM}z_{AM}, x_{BM}y_{BM}z_{BM} between the first and second images 237, 238, there is no need to the motion correction when the captured image data is reconstructed to the X-ray images.

Fig. 3 presents the functional parts of the unit 100.

The unit 100 comprises a control part 370 in order to control the unit 100, and its aforementioned movements and imaging processes.

The control part 370 comprises a processor part 372 in order to perform user and/or computer program (software) initiated instructions, and to process data.

The processor part 372 can comprise at least one processor.

In addition, the control part 370 can comprise a memory part 380 in order to store and to maintain data. The data can be instructions, computer programs, and data files.

The memory part 380 can comprise at least one memory.

If the processor part 372 comprises several processors, the processors can locate merely in the unit 100 or in at least one separate device, or so that one part of the processors locates in the unit 100 and another part of the processors locates in the at least one separate device that is configured to perform the formation or reconstruction of the image.

In addition, the control part 370 can comprise a data transfer part 374 in order to send control commands to at least one of the source part 124, detector part 126, the camera part 177, and a movement part 375.

The movement part 375 can comprise motors, drivers, or other parts 375 that cause the movements of at least one of the part 120, 124, 126, 128, 141, 162, 164, 166.

In addition, the data transfer part 374 can receive data from measuring parts or other detection parts that detect the function of the unit 100.

In addition, the data transfer part 374 can send control commands to at least one of the parts 124, 126, 177, 375.

In addition, the data transfer part 374 can receive information from at least one of the parts 124, 126, 177, 375.

In addition, the control part 370 can comprise a user interface part 178 in order to input control commands, to receive information and/or instructions, and to display information.

The UI part 178 can comprise at least one of a touchscreen, at least one function key, and a wired or wireless remote controller.

The UI part 178 can be attached to the column 140.

The memory part 380 can comprise at least a data transfer application 384 in order to control the data transfer part 374, a user interface application 388 in order to control the UI part 178, and a computer program (code) 389 in order to control the function of the unit 100.

The computer program 389 can control at least one of the movement part 375, detection devices, the source part 124, the detector part 126, and the camera part 177.

In addition, the computer program 389 can control imaging parameters, imaging sizes, and imaging modes.

The memory part 380 and the computer program 389, with the processor part 372, can cause the unit 100 at least to provide actions presented in context of the figures.

Such action can be taking, by the camera part 177, at least one image presenting the bite part 230 when it is arranged so that the location 205 of its end 233 is on the area 204 in the mouth 205 of the patient 201.

In addition, such action can be a calculation of locations and distances of the markers 231, 232 with respect to the unit 100 in the 3D coordinates from the taken at least one image.

In addition, such action can be a determination of the location 205 with respect to the unit 100 on a grounds of the calculated locations and distances of the markers 231, 232, and at least one known dimension of the bite part 230.

In addition, such action can be a determination of the area 204 on a grounds of the determined location 205 so that the unit 100 can perform the Panoramic, CT, or Cephalometric imaging of the area 204.

In addition, such action can be, after the area 204 has been determined, driving (moving) the rotating part 120 by means of at least one of the above presented movements to a position where the irradiation of the patient 201 will be started in order to provide the Panoramic, CT, or Cephalometric image from the area 204.

In addition, such action can be, after the rotating part 120 has been driven to the starting position, irradiating by the rotating part 120 the determined area 204 by means of at least one of the above presented movements, which is typical to the used Panoramic, CT, or Cephalometric imaging mode and which results the Panoramic, CT, or Cephalometric depending on the used imaging mode from the area 204.

In addition, such action can be a correction of a motion of the patient 201 during the irradiation.

The computer program 389 can be a computer program product that comprises a tangible, non-volatile (non-transitory) computer-readable medium bearing a computer program code 389 embodied therein for use with a computer (control part 370).

There is no need for significant accuracy how the patient 201 is positioned related to the lower shelf 142, when using the bite part 230, because the unit 100 has always right coordinates of the desired imaging volume.

In addition, the presented arrangement can be used for image stabilizer, when the position of the bite part 230 is observed with at least one camera 177 in real time during the irradiation. It does not matter whether the patient 201 moves or not during the scan. When the location of the patient 201 is known in every image frame, the motion of the patient 201 can be compensated (corrected) with the computer program 389 in order to obtain shaper images.

In addition, there is no need for rigid patient positioning at all, when using the bite part 230. The parts 142, 143 can be much lighter and more simple.

In addition, there are several adjusting methods for the parts 142, 143 to find appropriate angle for the head of the patient 201 with traditional patient supporting. There is no need for adjustments any more with the bite bite part 230, because it is sufficient to just point and shoot, and a location of the FOV is optimal in every irradiation.

## Claims

1. A portable bite stick (230) for determining an imaging area (204) of a patient (201) in panoramic, computed tomography, or cephalometric X-ray imaging, comprising
a first end (233) and
a second end (234),
which bite stick has known dimensions and
which first end is configured to be positioned in a mouth (202) of the patient,
**characterized in that** the bite stick further comprising
markers (231, 232),
which first end is configured to indicate the area inside the mouth,
which markers are configured to be positioned outside the mouth, and
which markers make possible to recognize positions of the markers, whereupon it is possible to calculate a location (205) of the first end with respect to an X-ray imaging unit (100) by using the positions of the markers and the known dimensions of the bite stick.

2. The bite stick of the preceding claim, which markers comprise at least first marker (231) and a second marker (232), wherein the second marker locates between the first and second ends, and wherein the first marker locates on the second end or between the second end and the second marker.

3. The bite stick of any of the preceding claims, which dimensions comprise at least one of a length of the bite stick, a distance between the first end and the first marker, a distance between the first and second markers, and at least one dimension of the first and second markers.

4. The bite stick of claim 3, which at least one dimension of the markers is a diameter, shape, or size of the markers.

5. The bite stick of any of the preceding claims, which markers comprise graphical symbols on a surface (235) of the bite stick, wherein the symbols comprise at least one of square, triangle, sphere, ring, and crossing line.

6. A method for using the portable bite stick (230) of any of the preceding claims for determining an imaging area (204) of a patient (201) in panoramic, computed tomography, or cephalometric X-ray imaging, comprising steps of
positioning a first end (233) of the bite stick in a mouth (202) of the patient so that a location (205) of the first end is in the area,
taking, by means of a camera part (177) of an X-ray imaging unit (100), at least one image presenting the bite stick, which comprises markers (231, 232), and
recognizing, by means of a processor part (372) of the unit, positions of the markers from the at least one image in order to calculate the location of the first end with respect to the unit by using the positions of the markers and the known dimensions of the bite stick.

7. The method of claim 6, wherein the camera part comprises an optical camera (177) that is mounted in a column (140) or a rotating unit (120) of the unit.

8. The method of claim 6, wherein the camera part comprises an X-ray source of an X-ray source part (124) of the unit and an X-ray imaging detector part (126) of the unit that are mounted in the rotating part, and wherein the at least one image comprises at least one X-ray image.

9. The method of any of claims 6-8, wherein the processor part calculates locations and distances of the markers with respect to the unit from the at least one taken image, and determines the location of the first end on a grounds of the calculated locations and distances, and the length of the bite stick.

10. The method of any of claims 6-9, wherein the camera part in the rotating unit takes two images and the processor part determines the location of the first end by using the two taken images.

11. The method of claim 6, wherein the camera part comprises two cameras (177) that are mounted in at least one of a carriage part (145) of the unit, the upper shelf, and the rotating unit.

12. The method of claim 11, wherein both cameras take the at least one image, and the processor part calculates the locations and distances of the markers from the taken images, and determines the location of the first end on a grounds of the calculated locations and distances, and the length of the bite stick.

13. A computer program (389) for determining an imaging area (204) of a patient (201) in panoramic, computed tomography, or cephalometric X-ray imaging, configured to perform the method of any of claims 6-12 and comprsing
an imaging code for taking, by means of a camera part (177) of an X-ray imaging unit (100), at least one image presenting a portable bite stick (230), which comprises markers (231, 232), and
a recognizing code for recognizing, by means of a processor part (372) of the unit, positions of the markers from the at least one image in order to calculate a location (205) of a first end (233) of the bite stick with respect to the unit by using the positions of the markers and known dimensions of the bite stick..

14. A tangible non-volatile computer-readable medium comprising the computer program (389) of claim 13.
